# EUROPÄISCHE PATENTSCHRIFT

(11) **EP 2 346 410 B1**
(45) Veröffentlichungstag und Bekanntmachung des Hinweises auf die Patenterteilung: **30.03.2016**
(21) Anmeldenummer: 09741200.1
(22) Anmeldetag: 08.09.2009
(51) Int. Cl.: A61B 10/02, G01N 33/53, A61B 10/04

(54) **BIOPSIE-INSTRUMENT ZUR ANREICHERUNG VON PROBENMATERIAL**
BIOPSY INSTRUMENT FOR ENRICHING SAMPLE MATERIAL
INSTRUMENT DE BIOPSIE POUR L ENRICHISSEMENT D UN MATÉRIAU ÉCHANTILLON

(30) Priorität: 08.09.2008 DE 102008046635; 04.06.2009 DE 102009024134
(43) Veröffentlichungstag der Anmeldung: 27.07.2011
(73) Patentinhaber: OGENO GMBH, 14195 Berlin (DE)
(72) Erfinder: PISON, Ulrich, 14163 Berlin (DE); MEISSNER, Wolfgang, 12209 Berlin (DE)
(74) Vertreter: Lange, Sven
(86) Internationale Anmeldenummer: PCT/DE2009/001268
(87) Internationale Veröffentlichungsnummer: WO 2010/025719

(56) Entgegenhaltungen:
- WO-A1-2009/147081
- WO-A2-2008/110392
- US-A- 5 983 899
- US-A1- 2003 049 679
- US-A1- 2007 003 989
- US-A1- 2008 213 130

## Beschreibung

Die vorliegende Erfindung bezieht sich auf ein Biopsie-Instrument nach Anspruch 1.

In der modernen Medizin wird die Biopsie, d.h. das Entfernen von Material aus einem lebenden menschlichen oder tierischen Körper zwecks Untersuchung mittels verschiedener Methoden wie beispielsweise der Mikroskopie, routinemäßig durchgeführt. Typischerweise wird ein derartiges Verfahren für die Diagnose von malignen und prämalignen Zelltransformationen, welche auf die Anwesenheit von kanzerösen oder präkanzerösen Tumoren hinweisen, verwendet. Die als Kembiopsien bekannten Routine-Techniken, bei welchen ganze Gewebesegmente zur histologischen Untersuchung entnommen werden, machen von verschiedenen Arten von hohlen nadelförmigen Biopsie-Instrumenten Gebrauch. Diese Instrumente weisen Kanülen mit einer scharfen frontseitigen Kante oder ein gekerbtes Stilett auf, um beim Einführen in den ausgewählten Körperbezirk in das Gewebe zu schneiden. Das als Biopsieprobe zu entfernende Gewebestück wird in der zylindrischen Bohrung der Kanüle aufgenommen. Beim Herausziehen des Biopsie-Instrumentes aus dem Körper wird die Probe mittels mechanischer Mittel oder Saugmittel in der zylindrischen Bohrung bewahrt und wird beim Herausziehvorgang vom Hauptkörper des Gewebes getrennt. Die entnommenen Proben haben typischerweise eine generell längliche zylindrische Form oder eine longitudinal halbzylindrische Form. Die Qualität, z. B. die Breite, die Länge und der Anteil an zerquetschten Zellen von Biopsieproben zur zytologischen oder histologischen Untersuchung ist ein wichtiger Faktor, welcher das Untersuchungsresultat beeinflusst. Gewebebiopsieproben sollten die Struktur des lebenden Gewebes so genau wie möglich widerspiegeln. Dementsprechend sollten Spannungen auf die Proben beim Exzidieren und Abtrennen vom Gewebe sowie beim Entfernen aus der Kanüle vermieden werden.

Die Entnahme von Material aus einem lebenden menschlichen oder tierischen Körper für nachgeschaltete Diagnostikverfahren erfolgt üblicherweise durch die Gewinnung einer soliden Gewebeprobe durch eine Biopsie oder durch die Entnahme von Körperflüssigkeiten, z.B. in Form einer Gefäßpunktion zur Gewinnung einer Blutprobe oder durch Pleurapunktion zur Gewinnung eines Pleuraergusses oder durch Gelenkpunktion zur Gewinnung von Synovialflüssigkeit oder durch Spinalkanalpunktion zur Gewinnung von Liquor oder generell durch die Punktion eines Körperhohlsystems zur Gewinnung von Material in flüssiger oder flüssigkeits-ähnlicher Form. Die vorliegende Erfindung beschreibt ein Biopsie-Instrument, welches in überraschend einfacher Weise ermöglicht, Zell- oder Molekülproben in verschiedenen Körperhohlsystemen von Mensch und Tier vor der Entnahme anzureichern, um eine ausreichende Konzentration für nachgeschaltete Diagnostikverfahren zu gewährleisten. Basierend auf diesem Biopsie-Instrument lassen sich demnach technische Lehren und Leitlinien formulieren, die neue oder verbesserte diagnostische Verfahren beschreiben.

Das US-Patent 5,282,476 beschreibt ein automatisiertes Gerät für die Biopsie, in welchem die Kanüle über einem Stilett angebracht ist, das die Kanüle zur Biopsiestelle lenkt. Nach dem Einführen wird die Kanüle über das Stilett hinaus getrieben und entnimmt dabei die Probe. Saugmittel werden zum Festhalten der Probe verwendet. Allerdings hat dieses Biopsie-Instrument wegen der Saugmittel einen ziemlich komplexen Aufbau und erfordert, beträchtliche Fähigkeiten bei der Handhabung. In den Händen eines ungeübten Anwenders kann das Instrument ungewisse Resultate ergeben. Ferner ist es für die Entnahme von Material aus soliden Körperstrukturen und nicht aus Körperhohlsystemen konzipiert.

Des Weiteren sind Instrumente oder Sensoren bekannt, die der Anreicherung von Proben dienen. Die WO/2006/131400 beschreibt die Nutzung einer funktionalisierten Oberfläche für die Anreicherung und Gewinnung von Molekülen oder seltenen Zellen, wie z.B. von fötalen Trophoblasten aus dem mütterlichen Blutkreislauf. Hierfür sind Rezeptorstrukturen auf der Oberfläche des Sensors verankert, die mit spezifischen Zellen oder Molekülen im Blutkreislauf reagieren und somit eine Anreicherung dieses Materiales *in situ* vor der Entnahme aus dem Körper gestatten sollen. Allerdings wurde bisher keine Ausgestaltung eines für diese Aufgabe tauglichen Biopsie-Instrumentes beschrieben, um die Herstellung für den Einsatz bei Mensch und Tier zu ermöglichen.

Weiterhin sind im Stand der Technik Vorrichtungen beschrieben (beispielsweise WO 01/23031 A1, EP 1 779 816 A2, US 2003/0135153, WO 98/22022 A1), mit deren Hilfe bestimmte Wirkstoffe oder Farbstoffe in Körperhöhlen einbringbar sind. Hierbei werden die Wirkstoffe in Aushöhlungen der Vorrichtungen eingebracht und beim Erreichen der Zielposition freigegeben. Nachteilig hierbei ist, dass die Proben sehr ungenau abgegeben werden und die Wirkstoffe in flüssiger Form in die Vorrichtungen einzubringen sind. Hierdurch sind die Einsatzmöglichkeiten der Vorrichtungen stark eingeschränkt.

Die im Stand der Technik beschriebenen Mittel zu der Entnahme oder Anreicherung von Proben sind nicht in allen Körperregionen einsetzbar. Insbesondere ist die endoskopische Verwendung beschriebener Instrumente/Sensoren nicht möglich oder nur schwer durchführbar. Andere wichtige Nachteile sind die Genauigkeit, mit welcher der für die Biopsie ausgewählte Gewebebezirk insbesondere Körperhohlsysteme mit dem Instrument/Sensor getroffen werden kann, die Einfachheit, mit welcher das Instrument/Sensor gehandhabt werden kann, die dem Patienten durch das Verfahren zugeführte Verletzung sowie die Kosten des Instrumentes oder des Sensors. Außerdem ist kein Biopsie-Instrument beschrieben, dass für den Einsatz bei Mensch und Tier konzipiert ist, um vor der Probenentnahme aus Körperhohlsystemen eine Anreicherung des Biopsiematerials risikoarm zu ermöglichen.

Ausgehend von diesem Stand der Technik ist es die Aufgabe der vorliegenden Erfindung, ein Biopsie-Instrument bereitzustellen, das die spezifische Anreicherung von Probenmaterial auch mittels endoskopischer Mittel und aus Körperhohlsystemen ermöglicht und über die Verweildauer im Körper nachgeschaltete diagnostische Verfahren erlaubt. Eine weitere Aufgabe der Erfindung ist die Entwicklung eines Biopsie-Instrumentes, das die Verletzungsgefahr für den Patienten minimiert, da besonders die Verweildauer des Biopsie-Instrumentes innerhalb des Körpers für das diagnostische Verfahren ein neues Kriterium bei der Probengewinnung ist.

Überraschenderweise wird diese Aufgabe durch die Merkmale der unabhängigen Ansprüche gelöst. Bevorzugte Ausführungsformen der Erfindung ergeben sich aus den Unteransprüchen.

Es war völlig überraschend dass das erfindungsgemäße Problem mit einem Biopsie-Instrument gelöst werden kann, welches folgende Komponenten umfasst:
i. ein Führungselement, umfassend einen feder-lastischen distalen und einen proximalen Bereich,
ii. ein Biofunktionselement, welches zwischen dem distalen und dem proximalen Bereich des Führungselement angebracht ist, und dessen Oberfläche Detektionsmoleküle aufweist, und
iii. ein mit dem proximalen Bereich des Führungselementes verbundenes Stabilisierungselement.

Das Biopsie-Instrument wird zur Anreicherung von Probenmaterial genutzt. Hierbei kann es sich um eine Probe aus der Gruppe umfassend Blut bzw. Blutplasma, Chylus bzw. Lymphe, Urin, Sperma, Vaginalsekret, Fruchtwasser, Speichel, Magensaft, Gallenflüssigkeit, Pankreassekret, Nasensekret, Bronchialsekret, Alveolarflüssigkeit, Liquor, Endolymphe, Kammerwasser, Tränenflüssigkeit, Synovialflüssigkeit, Pleuralflüssigkeit, Perikardflüssigkeit (Liquor pericardii), Peritonealflüssigkeit, Muttermilch, Schweiß, Menstruationsflüssigkeit oder Kombination hieraus handeln.

Das Biopsie-Instrument besteht mindestens aus einem Führungselement, einem Biofunktionselement und einem Stabilisierungselement. Das Führungselement weist einen distalen und einen proximalen Bereich auf, wobei der distale Bereich feder-elastisch ist.

Die Begriffe "proximal" und "distal" beziehen sich auf die Person, welche eine Biopsieprobe entnimmt. Dementsprechend ist das proximale Ende des Biopsie-Instrumentes das rückwärtige Ende, das vom Patienten weg weist.

Der Begriff feder-elastisch beschreibt die Eigenschaft eines Materials, sich unter Belastung zu verformen und bei Entlastung wieder die im Wesentlichen ursprüngliche Gestalt anzunehmen. Durch diese Eigenschaft des distalen Bereichs des Führungselementes wird vorteilhafterweise sichergestellt, dass eine geringe bis keine Verletzungsgefahr für den Patienten besteht, da sich der feder-elastische Bereich bei Kontakt mit einer Oberfläche, wie z.B. einer Gefäßwand oder einem Organ, verformt und somit der Druck auf die Oberfläche reduziert wird.

Zwischen dem distalen und dem proximalen Bereich des Führungselementes ist ein Biofunktionselement angebracht. Das Biofunktionselement weist auf seiner Oberfläche Detektionsmoleküle auf, die mittels chemischer, elektrochemischer und/oder biologischer Verfahren auf dessen Oberfläche aufgebracht sind. Detektionsmoleküle sind dem Fachmann ebenfalls als Fängermoleküle bekannt. Hierbei handelt es sich um Moleküle, die bevorzugt mit organischen und anorganischen Materialien interagieren. Als organische Materialien können z.B. Polymere verwendet werden. Etwa 90 % der organischen Materialien bestehen aus Kohlenstoff, Wasserstoff und Sauerstoff in wechselnden Mengenverhältnissen. Der überwiegende Rest organischer Verbindungen kann auch Stickstoff, Schwefel, Phosphor und Halogene enthalten. Des Weiteren können auch metallorganische Materialien, bestehend aus einer Verbindung aus Metall und Kohlenstoff verwendet werden. Als anorganische Materialien, d.h. nicht im Wesentlichen aus Kohlenstoff-bestehend, können Materialien umfassend Glasarten, Keramik, Hartstoffe, nanostrukturierte und nanokristalline, Silicium oder Silikate eingesetzt werden. Somit kann bedingt durch die spezielle Ausgestaltung des Biofunktionselementes eine Anreicherung von speziellen Zellen oder Molekülen im Körper von Mensch und Tier, d.h. *in situ,* erfolgen, was eine Abkehr vom technisch Üblichen darstellt und ein lang bestehendes Problem des Stands der Technik löst. Bedingt durch eine hohe Spezifität des Biofunktionselementes wird eine schnelle Anreicherung von Probenmaterial erreicht, wodurch der Patient geschont und entlastet wird.

In dem proximalen Bereich des Führungselementes befindet sich das Stabilisierungselement, welches mit diesem Bereich fest verbunden oder frei verschiebbar gestaltet sein kann. Das Stabilisierungselement fixiert das Biofunktionselement während der Biopsie und verhindert so eine Dislokation des Biofunktionselementes. Auch wird durch die erfindungsgemäße Ausführungsform des Biopsie-Instrumentes eine Entfernung des Instrumentes aus dem Körper des Patienten als Ganzes garantiert, d.h. durch die Anordnung der Komponenten wird eine Dislokation einzelner Komponenten verhindert, wodurch keine Komponente des Instrumentes im Körper des Patienten verbleibt.

Die erfindungsgemäße Lehre beschreibt ein Biopsie-Instrument, welches in überraschend einfacher Weise ermöglicht, Zell- oder Molekülproben in verschiedenen Körperhohlsystemen von Mensch und Tier vor der Entnahme anzureichern, um eine ausreichende Konzentration für nachgeschaltete Diagnostikverfahren zu gewährleisten.

Vorteile des erfindungsgemäßen Biopsie-Instrument sind die Genauigkeit mit welcher das Instrument in der für die Anreicherung von Probenmaterial ausgewählten Körperregion positioniert werden kann, die Spezifität und Sensitivität mit der sich Zellen und Moleküle *in situ* anreichern lassen und die Einfachheit, mit welcher das Biopsie-Instrument gehandhabt werden kann. Auch sind die Kosten für das Biopsie-Instrument gering. Eine schnelle und verlässliche Anreicherung von Proben zur Analyse, spielt in der Medizin eine wichtige Rolle und ist für die Diagnose von Krankheiten ein entscheidendes Kriterium.

Vorzugsweise sind die Komponenten des Biopsie-Instrumentes sequentiell von distal nach proximal angeordnet, nach dem distalen federelastischen Führungselement ist das Biofunktionselement angeordnet, wobei sich in dem proximalen Bereich des Führungselementes das Stabilisierungselement befindet. Durch diese Anordnung wird sichergestellt, dass bei der Einfuhr des Biopsie-Instrumentes in den Körper der feder-elastische Bereich distal angeordnet ist und somit eine Verletzungsgefahr für den Patienten minimiert wird. Das Biofunktionselement wird weiterhin von dem proximal gelegenen Stabilisierungselement fixiert. Somit wird eine Dislokation der Komponenten während der Probenanreicherung verhindert und ein Verlust einer Komponente des Biopsie-Instrumentes verhindert. Außerdem wird durch die sequentielle Anordnung der Komponenten die Zuverlässigkeit erhöht und die Effizienz einer Biopsie verbessert. Einem kundiger Fachmann ist es möglich Sonderformen des Biopsie-Instrumentes zu konstruieren, bei denen zwei oder mehr Biofunktionselementen mit unterschiedlichen Detektionsmolekülen zwischen distalem und proximalem Anteil des Führungselementes angeordnet sind oder die Funktionalisierung des distalen Anteiles des Führungselementes mit Detektionsmolekülen stattfindet unter Erhalt der feder-elastische Materialeigenschaften dieser Region.

Es ist weiterhin bevorzugt, wenn der distale Bereich des Biopsie-Instrumentes, also der feder-elastische Anteil des Führungselementes, aus metallischen oder nicht-metallischen Materialien gefertigt ist. Dementsprechend kann es in einer bevorzugten Ausführungsform aus rostfreiem Stahl aber auch anderen Metallen gefertigt werden. Metalle bezeichnen chemische Elemente, die sich im Gegensatz zu den Nichtmetallen im Periodensystem links der diagonalen Trennungslinie beginnend mit dem Element Beryllium (2. Gruppe) bis hin zum Polonium (16. Gruppe) befinden, sowie deren Legierungen und intermetallische Verbindungen (umfassend Laves-Phasen, Heusler-Phasen, Zintl-Phasen, Hume-Rothery-Phasen, NiTi, Co5, Nb3Sn oder Ni3Al) mit charakteristischen metallischen Eigenschaften. Metalle umfassen Aluminium, Beryllium, Bismut, Blei, Cadmium, Chrom, Eisen, Gallium, Gold, Indium, Kalium, Cobalt, Kupfer, Magnesium, Mangan, Molybdän, Natrium, Nickel, Osmium, Palladium, Platin, Quecksilber, Rhodium, Ruthenium, Silber, Tantal, Titan, Uran, Vanadium, Wolfram, Zink, Zinn oder Zirconium. In einer vorteilhafter Ausführungsform kann ein innerer Draht von einem äußeren Draht zirkulär umgeben sein, so dass sich die feder-elastische Eigenschaft einstellt.

Alternativ können für diese Komponente aber auch nichtmetallische Materialien, aus denen sich dünne zylindrische Strukturen formen lassen, verwendet werden. Ein Zylinder ist im Sinne der Erfindung eine Fläche, die von zwei parallelen, ebene Flächen (Grund- und Deckfläche) und einer Mantel- bzw. Zylinderfläche, die von parallelen Geraden gebildet wird, begrenzt ist. Nichtmetalle sind gasförmige (umfassend Wasserstoff, Stickstoff, Sauerstoff, Fluor, Chlor oder Edelgase), flüssige (umfassend Brom) oder feste (umfassend Bor, Kohlenstoff, Phosphor, Schwefel, lod, oder Astat) Elemente von meist elektronegativem Charakter. Nichtmetalle sind in den Hauptgruppen (Wasserstoff in der 1., die übrigen in der 13. -18. Gruppe) des Periodensystems zu finden.

Für nicht-metallische Materialien können in einer bevorzugten Ausführungsform Polymere verwendet werden. Polymere bezeichnen, nach einer Definition der IUPAC ("The International Union of Pure and Applied Chemistry") eine Substanz, die sich aus einem Kollektiv chemisch einheitlich aufgebauter, sich in der Regel aber hinsichtlich Polymerisationsgrad, Molmasse und Kettenlänge unterscheidender Makromoleküle (Polymermoleküle) zusammensetzt. Bei solchen sogenannten polymereinheitlichen Stoffen sind also alle Makromoleküle gleich aufgebaut und unterscheiden sich lediglich durch ihre Kettenlänge (Polymerisationsgrad). Man kann derartige Polymere als Polymerhomologe bezeichnen. Polymere können aus der Gruppe umfassend anorganische Polymere, metallorganische Polymere voll- oder teilaromatischen Polymeren, Homopolymere, Copolymere, Biopolymere, chemisch modifizierte Polymere und/oder synthetische Polymere ausgewählt werden und umfassen Polyethylen, Polypropylen, Polyvinylchlorid, Polystyrol, Polymethylmethacrylat, Polyamid, Polyester, Polycarbonat, Polyethylenterephthalat, Polyethylenglykol, Dendrimere, Silikone, Proteine, DNA, RNA, Kohlenhydrate oder Polyhydroxxyalkanoate. Bevorzugt wird Polytetrafluorethylen verwendet. Der aus diesen Materialien gefertigte distale Bereich des Biopsie-Instrumentes stellt einen technischen Fortschritt dar, da durch die bevorzugte Ausführungsform die Verletzungsgefahr reduziert wird. Das Material weist feder-elastische Eigenschaften auf und verhindert so eine Verletzung der Oberflächen mit denen das Biopsie-Instrument in Kontakt kommt.

Weiterhin vorteilhaft ist, dass das Stabilisierungselement des Biopsie-Instrument im Wesentlichen zylindrisch ausgebildet ist. Durch die vorteilhafte Ausführungsform kann das Stabilisierungselement auf den proximalen Bereich des Führungselementes einfach aufgebracht werden, indem es auf den proximalen Bereich aufgeschoben wird. Des Weiteren wird durch die vorteilhafte zylindrische Form eine leichte Einfuhr und Ausfuhr des Biospie-Instrumentes erreicht und somit die Effektivität gesteigert.

Vorteilhaft ist ebenfalls, dass das Stabilisierungselement über den proximalen Teil des Führungselementes schiebbar ist. Das Stabilisierungselement kann über den proximalen Bereich des Führungselementes aufgebracht werden, ohne die weiteren Komponenten, im Wesentlichen das Biofunktionselement, entfernen zu müssen. Das Stabilisierungselement fixiert das Biofunktionselement, was durch eine passgenaue Anfertigung der Komponente erreicht wird. Passgenau im Sinne der Erfindung bezeichnet, dass mindestens zwei Komponenten derart gefertigt sind, dass sie kompatibel sind und exakt in die für sie vorgesehene Position passen. Die Einfachheit, mit der das Stabilisierungselement auf den proximalen Bereich des Führungselementes aufgebracht wird, erleichtert die Arbeit des Fachmanns. Des Weiteren kann das Stabilisierungselement nach erfolgreicher Biopsie einfach entfernt werden und die angereicherten Proben des Biofunktionselementes weiter verarbeitet oder an entsprechende Bearbeitungsstellen weitergeleitet werden. Die vorteilhafte Ausführungsform stellt somit einen glücklichen Griff dar, da aus einer Vielzahl von Möglichkeiten eine bestimmte gewählt wurde, deren Ergebnis nicht vorausgesagt werden konnte, daher handelt es sich um ein patentwürdigen glücklichen Griff.

Vorteilhafterweise ist weiterhin vorgesehen, dass der Außendurchmesser des Stabilisierungselementes bevorzugt gleich dem Außendurchmesser des distalen Bereichs des Führungselementes ist. Hierdurch wird eine ebene, d.h. eine plane Oberfläche des Biopsie-Instrumentes geformt, wodurch eine Verletzung des Patienten stark reduziert ist. Des Weiteren wird durch die vorteilhafte Ausführungsform eine leichte Einfuhr des Biopsie-Instrumentes in Körperregionen garantiert und zusätzlich sichergestellt, dass es als Ganzes wieder entfernt wird. Im Ganzen bedeutet, dass durch die Anordnung der Komponenten eine Dislokation einzelner Komponenten verhindert wird, wodurch keine Komponente des Instrumentes im Körper des Patienten verbleibt.

Eine weitere vorteilhafte Ausführungsform umfasst ein Biopsie-Instrument wobei das Stabilisierungselement reversibel mit dem proximalen Bereich des Führungselementes verbunden ist. Vorzugsweise lässt sich das Stabilisierungselement gemäß dieser Ausführungsform, nach der Biopsie von dem proximalen Bereich des Führungselementes wieder entfernen, um das Biofunktionselement freizugeben. Das Biofunktionselement kann zur Durchführung von nachgeschalteten Diagnose-Verfahren in die entsprechenden Bearbeitungsstellen versendet werden oder es kann eine Analyse der angereicherten Proben vor Ort vorgenommen werden. Die vorteilhafte Ausführungsform stellt einen technischen Fortschritt dar, da der Fachmann das Biopsie-Instrument einfach zusammenbauen kann und es nach beendeter Probenanreicherung wieder in die einzelnen Komponenten zerlegen kann. Das Stabilisierungselement kann so mehrmals, nach entsprechender Desinfektion, genutzt werden, wodurch die Kosten reduziert werden.

Weiterhin ist vorteilhaft, wenn das Stabilisierungselement vorzugsweise aus Kunststoff gefertigt ist. Kunststoffe bezeichnen Materialien, deren wesentliche Bestandteile aus solchen makromolekularen organischen Verb. bestehen, die synthetisch oder durch Abwandeln von Naturprodukten entstehen. Sie sind in vielen Fällen unter bestimmten Bedingungen (Wärme u. Druck) schmelz- und formbar. Zu den Kunststoffen gehören auch die Kautschuke und die Chemiefasern. Auch die synthetischen Lackrohstoffe und die Klebstoffe können zu den Kunststoffen gezählt werden. Für die vorteilhafte Ausführungsform können Kunststoffe aus der Gruppe abgewandelte Naturstoffe, synthetische Kunststoffe (Polykondensate, Polymerisate, Polyaddukte), Duroplaste, und/oder ungesättigte Polyesterharze, umfassend Cellulosenitrat, Celluloseacetat, Cellulosemischester, Celluloseether, Polyamid, Polycarbonat, Polyester, Polyphenylenoxid, Polysulfon, Polyvinylacetal, Polyethylen, Polypropylen, Poly-1-buten, Poly-4-methyl-1-penten, Ionomere, Polyvinylchlorid, Polyvinylidenchlorid, Polymethyl-methacrylat, Polyacrylnitril, Polystyrol, Polyacetal, Fluor-Kunststoffe, Polyvinylalkohol, Polyvinylacetat, Poly-*p-*xylylen, lineare Polyurethane, chlorierte Polyether, Casein-Kunststoffe, Phenol-Harz, Hamstoff-Harz, Thiohamstoff-Harz, Melamin-Harz, Epoxidharz, vernetzte Polyurethane, Alkydharz, Allylharz, Silicon, Polyimid, und/oder Polybenzimidazol verwendet werden. Durch die Verwendung von Kunststoff bei der Ausgestaltung des Stabilisierungselementes, sind Biopsie-Instrumente mit gummi-elastischen Eigenschaften herstellbar, die sich an unterschiedlichen Biopsie-Orten im Körper platzieren lassen. Durch die vorteilhafte Ausführungsform kann somit eine erhöhte Zuverlässigkeit und Flexibilität bei der Durchführung einer Biopsie erreicht werden. Auch ist die Verletzungsgefahr des Patienten drastisch reduziert, da die gummi-elastische Ausführungsform keine Oberflächen verletzen. Des Weiteren können durch die Verwendung von Kunststoffen für die Ausgestaltung Kosten reduziert werden.

Vorteilhafterweise ist vorgesehen, dass das Stabilisierungselement und der proximale Bereich des Führungselementes mechanisch verbunden, verklebt und/oder verschweißt sind. Hierdurch wird eine stabile Verbindung des proximalen Bereichs des Führungselementes und des Stabilisierungselementes erreicht, wodurch die Handhabung des Biopsie-Instrumentes für den Fachmann erleichtert wird. Durch eine mechanische Verbindung kann die Verbindung reversibel gestaltet werden, wobei hierfür dem Fachmann bekannte Befestigungsmittel verwendet werden. Weiterhin sind dem Fachmann Verklebungen oder Verschweißungen bekannt, die eine sichere und permanente Verbindung des proximalen Bereichs und des Stabilisierungselementes sicherstellen. Durch die stabile Verbindung wird garantiert, dass das Biopsie-Instrument als Ganzes in einen Patienten eingeführt und auch nach Probenanreicherung wieder ausgeführt werden kann, wodurch die Zuverlässigkeit verbessert und der Biopsie-Prozess an sich vereinfacht wird.

Des Weiteren vorteilhaft ist, dass das Führungselement und das Stabilisierungselement in einem Herstellungsverfahren gefertigt sind, das heißt es können für eine Anwendung Materialien für beide Komponenten verwendet werden, die auf die entsprechenden Körperregionen in Größe und Form abgestimmt sind. Eine vorteilhafte Ausführungsform ist Kunststoff, aus der Gruppe abgewandelte Naturstoffe, synthetische Kunststoffe (Polykondensate, Polymerisate, Polyaddukte), Duroplaste, und/oder ungesättigte Polyesterharze, umfassend Cellulosenitrat, Celluloseacetat, Cellulosemischester, Celluloseether, Polyamid, Polycarbonat, Polyester, Polyphenylenoxid, Polysulfon, Polyvinylacetal, Polyethylen, Polypropylen, Poly-1-buten, Poly-4-methyl-1-penten, Ionomere, Polyvinylchlorid, Polyvinylidenchlorid, Polymethyl-methacrylat, Polyacrylnitril, Polystyrol, Polyacetal, Fluor-Kunststoffe, Polyvinylalkohol, Polyvinylacetat, Poly-*p*-xylylen, lineare Polyurethane, chlorierte Polyether, Casein-Kunststoffe, Phenol-Harz, Harnstoff-Harz, Thioharnstoff-Harz, Melamin-Harz, Epoxidharz, vernetzte Polyurethane, Alkydharz, Allylharz, Silicon, Polyimid, und/oder Polybenzimidazol. Hierdurch kann erreicht werden, dass die Verletzungsgefahr für den Patienten reduziert wird, da eine ebene Oberfläche des Instrumentes entsteht. Auch wird für den Fachmann die Handhabung des Biopsie-Instrumentes vereinfacht und die Herstellungskosten für das Biopsie-Instrument können gering gehalten werden.

In einer vorteilhaften Ausführungsform ist vorgesehen, dass das Biofunktionselement eine im Wesentlichen zylindrische Form aufweist, wobei auch andere geometrische Formen verwendbar sind, umfassend Polygon, Dreieck, Viereck, Fünfeck, Sechseck, Siebeneck, Achteck, Neuneck, Zehneck, Ellipse, Kreis, Hyperbel, Parabel, Superellipse, Kugeldreieck, Zykloide, Rosette, Helix, Kugel, Ellipsoid, Rotationsellipsoid, Paraboloid, Rotationsparaboloid, Hyperboloid, Oloid oder Polyeder. Durch die Ausführungsform kann das Biofunktionselement leicht von proximal auf das Führungselement aufgebracht werden, wobei es nach dem Aufbringen räumlich von dem federelastischen distalen Bereich und dem proximalen Stabilisierungselement fixiert wird. Hierdurch ist eine Dislokation des Biofunktionselementes ausgeschlossen. Des Weiteren wird durch die im Wesentlichen zylindrische Form sichergestellt, dass das Biopsie-Instrument eine ebene Oberfläche aufweist und eine leichte Einfuhr und Ausfuhr des Biopsie-Instrumentes garantiert werden kann. Somit kann durch die vorteilhafte Ausführungsform die Qualität der Probenanreicherung erhöht werden wobei die Zeit der Probenanreicherung reduziert wird.

Vorteilhafterweise hat das Biofunktionselement einen Außendurchmesser, der größer, gleich oder kleiner, vorzugsweise 0,01 bis 0,1 mm kleiner als der Außendurchmesser des distalen Bereichs des Führungselementes ist. Durch die vorteilhafte Ausführungsform wird erreicht, dass das Biofunktionselement von proximal auf das Führungselement aufgebracht werden kann, jedoch von dem distalen Bereich räumlich begrenzt wird. Der Außendurchmesser des Biofunktionselementes ist bevorzugt 0,01 bis 0,1 mm kleiner als der Außendurchmesser des distalen Bereichs des Führungselementes. Durch den bevorzugten Außendurchmesser des Biofunktionselementes kann eine nahezu ebene Oberfläche des Biopsie-Instrumentes erzeugt werden, wodurch überraschenderweise eine optimale Funktionalität des Instrumentes garantiert wird, da bei der Einfuhr und Ausfuhr des Biopsie-Instrumentes das Instrument nicht mit biologischen Oberflächen interagiert und so die Positionierung des Biopsie-Instrumentes nicht beeinträchtigt wird. Der Außendurchmesser des Biopsie-Instrumentes und demgemäß der Außendurchmesser des Biofunktionselementes orientiert sich an der anatomischen Struktur, in der das Biopsie-Instrument eingesetzt werden soll. Die Länge ist abhängig von der Punktionsstelle und der Positionierung für die Probensammlung *in situ.* Die Dimensionen des Biopsie-Instrument können einfach und schnell an die Anforderungen der unterschiedlichen Biopsie-Orte angepasst werden, woraus ein Ersparnis an Zeit und Kosten resultiert.

Weiterhin ist vorteilhafterweise vorgesehen, dass das Führungselement zwischen dem distalen und proximalen Bereich eine Aufnahmevorrichtung aufweist, welche der Aufnahme des Biofunktionselementes dient. Bei der Aufnahmevorrichtung kann es sich um Aussparungen verschiedener geometrischer Formen handeln, umfassend, Polygon, Dreieck, Viereck, Fünfeck, Sechseck, Siebeneck, Achteck, Neuneck, Zehneck, Ellipse, Kreis, Hyperbel, Parabel, Superellipse, Kugeldreieck, Zykloide, Rosette, Helix, Kugel, Ellipsoid, Rotationsellipsoid, Paraboloid, Rotationsparaboloid, Hyperboloid, Oloid oder Polyeder. Eine vorteilhafte Ausführungsform umfasst zylindrisch, halbrylindrisch, spangenförmig oder folienförmig Aussparungen. Hierbei handelt es sich um Beispiele, die keine Limitierung der Aufnahmevorrichtung darstellen sollen. Folienförmig bezeichnet im Sinne der Erfindung dass die Aufnahmevorrichtung oder das Biofunktionselement derart geformt sind, das sie eine sehr geringe Dicke und große Fläche aufweisen. Das Biofunktionselement wird entsprechend der Form der Aufnahmevorrichtung geformt. Auch diese Ausführungsaltemativen stellen sicher, dass durch die vorteilhafte Anordnung einer distal gelegenen Führungselementes gefolgt von einem Biofunktionselementes sich ein Stabilisierungselement anschließt. So wird erreicht, dass bei minimaler Verletzungsgefahr für den Patienten die Biofunktion des Instrumentes gewährleistet bleibt und sich das Biopsie-Instrument nach Sammlung von Probenmaterial *in situ* in Gänze von außen aus dem Körper wieder entfernen lässt. Des Weiteren kann durch die vorteilhafte Ausführungsform das Biopsie-Instrument an unterschiedliche Anwendungen angepasst werden, wobei auch durch die Form der Aufnahmevorrichtung bzw. des Biofunktionselementes die zu sammelnde Probenmenge variiert und an den Bedarf des Anwenders angepasst werden kann.

Vorteilhafterweise umfasst das Biofunktionselement Metall, Kunststoff, und/oder keramischen Werkstoffe. Metallen umfassen, wie bereits oben aufgeführt, Aluminium, Beryllium, Bismut, Blei, Cadmium, Chrom, Eisen, Gallium, Gold, Indium, Kalium, Cobalt, Kupfer, Magnesium, Mangan, Molybdän, Natrium, Nickel, Osmium, Palladium, Platin, Quecksilber, Rhodium, Ruthenium, Silber, Tantal, Titan, Uran, Vanadium, Wolfram, Zink, Zinn oder Zirconium. Keramischen Werkstoffe bezeichnen eine Sammelbezeichnung für aus anorganischen und überwiegend nichtmetallischen Verbindungen oder Elementen aufgebaute und zu mehr als 30 Volumen-% kristallisierte Materialien. Keramische Werstoffen umfassen, Tonerde bzw. Kaolin, Quarz, Feldspat, Kalk, Silimanit, Magnesit, Terrakotta, Majolika, Fayence, Raku, Paperclay oder Oxidkeramiken. Für die vorteilhafte Ausführungsform können des Weiteren Kunststoffe, wie bereits oben erwähnt, umfassend Cellulosenitrat, Celluloseacetat, Cellulosemischester, Celluloseether, Polyamid, Polycarbonat, Polyester, Polyphenylenoxid, Polysulfon, Polyvinylacetal, Polyethylen, Polypropylen, Poly-1-buten, Poly-4-methyl-1-penten, lonomere, Polyvinylchlorid, Polyvinylidenchlorid, Polymethyl-methacrylat, Polyacrylnitril, Polystyrol, Polyacetal, Fluor-Kunststoffe, Polyvinylalkohol, Polyvinylacetat, Poly-*p*-xylylen, lineare Polyurethane, chlorierte Polyether, Casein-Kunststoffe, Phenol-Harz, Hamstoff-Harz, Thiohamstoff-Harz, Melamin-Harz, Epoxidharz, vernetzte Polyurethane, Alkydharz, Allylharz, Silicon, Polyimid, und/oder Polybenzimidazol verwendet werden. Die vorteilhafte Ausführungsform stellt sicher, dass eine effektive und schnelle Probenanreicherung durch das Biofunktionselement erreicht wird und das gesammelte Material an dem Biofunktionselement gebunden wird. Außerdem wird durch die Wahl des Werkstoffes, die Verletzungsgefahr für den Patienten reduziert. Eine Vergrößerung der Oberfläche der Werkstoffe ist zusätzlich möglich, um die Effektivität der Probenanreicherung zusätzlich zu steigern.

In einer weiteren vorteilhaften Ausführungsform ist vorgesehen, dass die Detektionsmoleküle bevorzugt aus der Gruppe umfassend Antikörper, Antikörperfragmente, Peptide, Nukleinsäuren, Rezeptoren und/oder anorganischen Materialien ausgewählt sind. Das Aufbringen von Detektionsmolekülen auf dem Biofunktionselement erreicht eine spezifische Anreicherung von Proben *in situ* vor der Entnahme der Probe. Die Detektionsmoleküle können mit Zellen und/oder Molekülen im Körper von Mensch oder Tier reagieren und binden die Zellen und/oder Moleküle. Hierfür können unterschiedliche Detektionsmoleküle Verwendung finden und auch miteinander kombiniert werden. Durch die spezifische Positionierung des Biopsie-Instrumentes in einer gewünschten Körperregion und durch die Spezifität und Sensitivität des Instrumentes, kann erreicht werden, dass auch geringe Konzentrationen an Molekülen und/oder Zellen durch die Detektionsmoleküle detektiert werden. Dies stellt einen technischen Fortschritt dar. Des Weiteren ist durch eine Variation der Detektionsmoleküle ein breites Anwendungsfeld der vorteilhaften Ausführungsform denkbar. Es kann ebenfalls vorteilhaft sein, unterschiedliche Detektionsmoleküle auf das Biofunktionselement aufzubringen. So können beispielsweise unterschiedliche Detektionsmoleküle, die beispielsweise spezifisch für einen bestimmten Zelltyp sind, aufgebracht werden. Hierdurch wird sichergestellt, dass bevorzugt eine spezifische Probe angereichert wird. Die Moleküle oder Zellen werden schnell und effizient gesammelt und können anschließend vorteilhafterweise in einem Labor quantifiziert und analysiert werden. Besonders eine schnelle und zuverlässige Probenanreicherung spielt in der Medizin oder genauer der Diagnostik eine entscheidende Rolle für die Diagnose von Krankheiten.

Weiterhin ist vorteilhafterweise vorgesehen, dass das Biopsie-Instrument zusätzlich eine Abdeckvorrichtung aufweist. Die Abdeckvorrichtung hat vorzugsweise eine hülsenförmige Struktur, die das Biofunktionselement abdeckt. Andere geometrische Formen, umfassend Polygon, Dreieck, Viereck, Fünfeck, Sechseck, Siebeneck, Achteck, Neuneck, Zehneck, Ellipse, Kreis, Hyperbel, Parabel, Superellipse, Kugeldreieck, Zykloide, Rosette, Helix, Kugel, Ellipsoid, Rotationsellipsoid, Paraboloid, Rotationsparaboloid, Hyperboloid, Oloid oder Polyeder sind ebenfalls verwendbar. Dadurch lässt sich das Biofunktionselement und das dort nach der Anreicherung *in situ* lokalisierte Probenmaterial vor der Entnahme aus dem Körper vor Abscherung oder Deformation schützen und eine Kontamination außerhalb des Körpers vermeiden. Eine vorteilhafte Ausführungsform ist dass die Abdeckvorrichtung eine Schiebevorrichtung darstellt, die von dem proximalen Bereich über das Biofunktionselement geschoben wird. Jedoch sind auch andere Vorrichtungen, umfassend Klappvorrichtungen denkbar, die der Abdeckung des Biofunktionselement dienen. Durch die vorteilhafte Ausführungsform wird die angereicherte Probe geschützt, wodurch Messfehler reduziert werden und die Qualität der Anreicherung verbessert wird.

Vorteilhaft ist, dass die Abdeckvorrichtung vorzugsweise das Biofunktionselement abdeckt, wobei die Abdeckvorrichtung Homopolymere, Copolymere, Biopolymere, chemisch modifizierte Polymere und/oder synthetische Polymere umfasst. Als Materialien für die Abdeckvorrichtung eignen sich Polymermaterialien, umfassend Polyethylen, Polypropylen, Polyvinylchlorid, Polystyrol, Polymethylmethacrylat, Polyamid, Polyester, Polycarbonat, Polyethylenterephthalat, Polyethylenglykol, Dendrimere, Silikone, Proteine, DNA, RNA, Kohlenhydrate oder Polyhydroxxyalkanoate. Vorzugsweise allerdings Polytetrafluorethylen. Insbesondere wenn die angrenzenden Komponenten aus dem gleichen Material gefertigt sind, wird dadurch eine gute Verschiebbarkeit der Einzelkomponenten erreicht. Durch die vorteilhafte Ausführungsform kann die Abdeckvorrichtung leicht über das Biofunktionselement geschoben werden oder dieses abdecken. Durch die vorteilhafte Ausführungsform werden Fehler bei der Biopsie-Entnahme reduziert und so die Effizienz verbessert und der Arbeitsaufwand reduziert.

Eine weitere vorteilhafte Ausführungsform ist, dass das Biopsie-Instrument eine longitudinale Bohrung aufweist, zur Aufnahme bevorzugt eines Mandrains und/oder anderer Funktionselemente. Die Bohrung kann durch dem Fachmann bekannte Verfahren in das Biopsie-Instrument eingebracht werden und die Breite der Bohrung richtet sich nach der Anwendung und kann dementsprechend variieren. In den longitudinalen dünnen Bohrkanal kann z.B. ein Mandrain (ein Führungsdraht) eingebracht werden und bis in das distale Ende des Biopsie-Instrumentes vorgeschoben werden. So kann die Steifigkeit des Instrumentes für Platzierungsmanöver im Körper optimiert werden. Auch können in eine solche Bohrung weitere Funkfionalisierungen z.B. elektronischer Art eingebracht werden, um die Nutzbarkeit des Biopsie-Instrumentes zu erhöhten. Die longitudinale Bohrung verbessert die Arbeitssicherheit und stellt eine Vereinfachung dar.

Desweiteren sind Gestaltungsformen des Biopsie-Instrumentes möglich, die folgende Spezialfälle beinhalten aber nicht darauf beschränkt sind und durch den kundigen Fachmann leicht ausgeführt werden können: a) die Anordnung von zwei oder mehr Biofunktionselementen mit unterschiedlichen Detektionsmolekülen zwischen distalem und proximalem Anteil des Führungselementes; b) die Funktionalisierung des distalen Anteiles des Führungselementes mit Detektionsmolekülen unter Erhalt der feder-elastischen Materialeigenschaft dieser Region.

Es ist weiterhin Gegenstand der Erfindung, einen Biopsie-Kit zur Anreicherung von Probenmaterial aus unterschiedlichen Körperregionen und Körperhohlsystemen und dessen Verwendung zur Verfügung zu stellen. Das Biopsie-Kit umfasst mindestens die folgenden Komponenten des Biopsie-Instrumentes:
i. ein Führungselement, umfassend einen federelastischen distalen und einen proximalen Bereich,
ii. ein oder mehrere Biofunkfionselemente, welche zwischen dem distalen und dem proximalen Bereich des Führungselement angebracht sind, wobei auf der Oberfläche von mindestens einem Biofunktionselement Detektionsmoleküle aufgebracht sind, und
iii. ein mit dem proximalen Bereich des Führungselementes verbundenes Stabilisierungselement.
wobei das Führungselement, das Biofunktionselement und das Stabilisierungselement sequentiell von distal nach proximal angeordnet sind.

Das Biopsie-Kit umfasst mindestens ein Führungselement, ein Biofunktionselement und ein Stabilisierungselement, wobei das Führungselement, das Biofunktionselement und das Stabilisierungselement sequentiell von distal nach proximal angeordnet sind. Das Führungselement weist einen distalen und einen proximalen Bereich auf und der distale Bereich ist feder-elastisch gestaltet. Es kann ebenfalls vorteilhaft sein, Sonderformen des Biopsie-Instrumentes zu konstruieren, bei denen zwei oder mehr Biofunktionselemente mit unterschiedlichen Detektionsmolekülen zwischen distalem und proximalem Anteil des Führungselementes angeordnet sind. Vorteilhafterweise kann die Funktionalisierung des distalen Anteiles des Führungselementes mit Detektionsmolekülen erfolgen und die feder-elastischen Materialeigenschaften dieser Region erhalten bleiben. Ergänzt werden kann das Biopsie-Kit um spezifische Komponenten, die bevorzugt die Anwendung für spezielle Biopsie-Orte erleichtern (beispielsweise im Blutgefäßsystem oder im Urogenitalapparat) oder eine Anwendung unter Nutzung anderer medizintechnischer Hilfsmittel (z.B. von starren oder flexiblen Endoskopen) ermöglichen.

Die Begriffe "proximal" und "distal" beziehen sich auf die Person, welche eine Biopsieprobe entnimmt. Dementsprechend ist das proximale Ende eines Biopsie-Instrumentes das rückwärtige Ende, das vom Patienten weg weist. Der Begriff feder-elastisch beschreibt eine Eigenschaft eines Materials, dass sich unter Belastung verformt und bei Entlastung wieder die ursprüngliche Gestalt annimmt. Die Verletzungsgefahr des Patienten bei der Biopsie wird durch das Biospie-Kit, genauer durch die Gestaltung des Biopsie-Kit reduziert. Besonders der feder-elastische distale Bereich des Führungselementes stellt sicher, dass bei Kontakt mit einer Oberfläche, wie z.B. eine Gefäßwand oder ein Organ, keine Beschädigung des biologischen Materials erfolgt. Das feder-lastische Führungselement verformt sich, sobald der Druck auf die Oberfläche zu groß ist, wodurch der Druck auf die Oberfläche reduziert wird.

Zwischen dem distalen und dem proximalen Bereich des Führungselementes ist ein Biofunktionselement angebracht. Das Biofunktionselement dient der Aufnahme von Detektionsmoleküle, die auf dessen Oberfläche aufgebracht sind. Detektionsmoleküle sind Moleküle, die mit organischen und anorganischen Materialien interagieren. Somit kann bedingt durch die spezielle Ausgestaltung des Biofunktionselementes eine Anreicherung von speziellen Zellen oder Molekülen im Körper von Mensch und Tier, d.h. *in situ,* erfolgen, was eine Abkehr vom technisch Üblichen darstellt und ein lang bestehendes Problem des Stands der Technik löst. Bedingt durch die hohe Spezifität des Biofunktionselementes wird eine schnelle Anreicherung von Probenmaterial erreicht, wodurch der Patient geschont und entlastet wird.

Des Weiteren ist ein Stabilisierungselement mit dem proximalen Bereich des Führungselementes verbunden. Das Stabilisierungselement dient der Fixierung des Biofunktionselement während der Biopsie und verhindert so eine Dislokation des Biofunktionselementes. Somit wird erreicht, dass das Biopsie-Instrument als Ganzes aus dem Patienten entfernt wird, d.h. keine Komponente des Biopsie-Instrumentes interagiert mit Oberflächen derart, dass eine Beschädigung der Oberflächen verursacht wird und Komponenten des Instrumentes in den Körperregionen verbleiben. Vorteile des erfindungsgemäßen Biopsie-Kit sind die Genauigkeit mit der das Instrument in Körperregionen positioniert werden kann und die Spezifität und Sensitivität mit der sich Zellen und Moleküle *in situ* anreichern lassen. Das Biopsie-Kit ist leicht von einem Fachmann bedienbar und reduziert das Verletzungsrisiko für den Patienten. Weitere Vorteile sind die geringen Kosten des Biopsie-Kits und eine hohe Flexibilität bei der Ausgestaltung für unterschiedliche Biopsieorte und Anwendungsfälle auch unter Nutzung zusätzlicher Hilfsmittel wie z.B. Endoskopen. Des Weiteren ist eine schnelle und verlässliche Anreicherung von Proben zur Analyse für die Diagnose von Krankheiten ein entscheidendes Kriterium.

Es wird ein Biopsie-Kit bereitgestellt mit dem es gelingt, die für eine Probenanreicherung notwendigen Komponenten zu positionieren, stationär für die zur Anreicherung des spezifischen Probenmaterials erforderlichen Zeitspanne im Körper zu halten und nach der Verweilzeit im Körper mit dem gesammelten Probenmaterial in Gänze zu entfernen. Zur korrekten Positionierung für die Probensammlung und für die erfolgreiche Entnahme Prozedur des Probenmaterials aus dem Körper, dienen visuelle oder andere Führungsmarken an den Komponenten des Biopsie-Kits, die durch Gravur, farbliche oder taktile oder signalgebende Marken realisiert werden können und eine geografische Ortung des Biopsie-Instrumentes während der Positionierung und Probensammlung innerhalb des Körpers und für den Vorgang der Entfernung des Biopsie-Instruments aus dem Körper ermöglichen. Die Positionierung des Biopsie-Instrumentes kann mittels bildgebender Verfahren in situ sichergestellt werden. Hierzu sind dem Fachmann bekannte kontrastgebende Vektoren konstruktionsseitig zu verwenden. Auch kann das Biopsie-Kit hinsichtlich der Dimensionierung der Einzelkomponenten für spezielle Biopsie-Orte angepasst werden und um Hilfsmittel ergänzt werden, um für spezielle Anwendungsfälle notwendige oder wünschenswerte Bestandteile während der Biopsie verfügbar zu machen. Des Weiteren kann das Biopsie-Kit als Einmal-Artikel bereitgestellt werden, welches hygienischen Ansprüchen in medizinischen Einrichtungen besser erfüllt.

Das Biopsie-Kit kann vorteilhafterweise zur Entnahme von Proben vorzugsweise aus dem Blutgefäßsystem unter Einbeziehung der Herzkammern, des Lungen- und Körperkreislaufes und des arteriellen und venösen Systems als spezielle Biopsieorte, dem Gastrointestinaltrakt, wobei das Biopsie-Instrument wahlweise von oral oder anal positioniert werden kann, aus den ableitetenden Drüsengängen von Bauchspeicheldrüse, Tränendrüse, Ohrspeicheldrüse, aus den ableitetenden Drüsengängen von mükösen Drüsen, von gemischten Drüsen und von Haut- und Hautanhangsdrüsen einschließlich der Milchdrüse, aus dem Spinalkanal oder Himkammersystem, aus der Gallenblase und ihren ableitenden anatomischen Strukturen, aus den ableitenden Hamwegen oder dem lymphatischen System oder zur Entnahme von Proben aus den Körperhöhlen des Bauches oder des Brustkorbes, der Gebärmutter, des Urogenitalapparates oder eines Gelenkspaltes verwendet werden.

Eine flexible Gestaltung des Biopsie-Kits erlaubt die Anreicherung von Proben in unterschiedlichen Systemen. So kann das Biofunktionselement entsprechend den Anforderungen mit unterschiedlichen Detektionsmolekülen ausgestattet werden, die die Detektion system-spezifischer Proben erlaubt. Ein bevorzugtes Anwendungsgebiet ist das Blutgefäßsystem des Körpers. In diesem Fall wird das Biopsie-Kit über eine Blutgefäß-Verweilkanüle im Gefäßsystem positioniert. Weitere Anwendungsgebiete umfassen den Gastrointestinaltrakt, die ableitenden Gallengänge, den *ductus pancreaticus* oder die ableitenden Hamwege. Während für die Anwendung im Blutgefäßsystem der Außendurchmesser des Biopsie-Kits sich an dem Durchmesser des Blutgefäßes orientiert ist die Länge abhängig von der Punktionsstelle und der Positionierung für die Probensammlung *in situ.* Der Außendurchmesser bei Platzierung im *ductus pancreaticus, ductus choledochus, ductus cysticus* oder den ableitenden Hamwegen oder der Hamblase oder dem Peritoneum oder dem Tracheobronchialbaum oder der Pleurahöhle oder einem Gelenkspalt kann entsprechend angepasst werden. Die vorteilhafte Ausführungsform und Dimensionierung kann somit durch schnelle und einfache Adaptationen von dem Fachmann vorgenommen werden. Es löst demgemäß ein lange im Stand der Technik bestehendes Problem und stellt eine schnelle und spezifische Ausführungsform zur Verfügung, die in unterschiedlichen Körperregionen vorzugsweise in Körperhohlsystemen einsetzbar ist.

Ausführungsbeschreibungen des Biopsie-Kits erfolgen beispielhaft für Anwendungsfälle unter Nutzung eines Endoskops und für die Probenanreicherung und Entnahme aus dem Blutgefäßsystem als Hauptanwendungsfälle, die Zusammenstellung des Biopsie-Kits ist aber auf diese Einzelfälle nicht beschränkt, sondern kann durch den kundigen Fachmann für andere Anwendungsfälle geändert oder ergänzt werden.

Vorteilhaft ist die Verwendung eines Biopsie-Kits, wenn das Biopsie-Instrument über endoskopische Hilfsmittel, vorzugsweise Gastroduodenoskop, Cystoskop, Urteroskop, Rektoskop, Proktoskop, Coloskop, Arthroskop, Laparoskop, Kolposkop, Hysteroskop, Ophthalmoskop, Laryngoskop und/oder Bronchoskop platziert wird. Ein Endoskop ist im Sinne der Erfindung ein Gerät, mit dem das Innere von lebenden oder toten Organismen, untersucht oder manipuliert werden kann. Für den Gebrauch des Biopsie-Instrumentes über ein Endoskop sind folgende Bestandteile als Komponenten des Biopsie-Kits vorteilhaft:
i. ein Führungselement, umfassend einen feder-elastischen distalen und einen proximalen Bereich,
ii. ein Detektionsmoleküle aufweisendes Biofunktionselement, welches zwischen dem distalen und dem proximalen Bereich des Führungselement angebracht ist,
iii. ein mit dem proximalen Bereich des Führungselementes verbundenes Stabilisierungselement,
iv. ein Adapter, der das Einbringen des Biopsie-Instrumentes über den Arbeitskanal des Endokops ermöglicht und
v. eine transparente und verschiebbare Hülle, die auf den Adapter aufsteckbar ist, das Biopsie-Instrument außerhalb des Körpers umgibt und eine hygienische Handhabung während seiner Verweilzeit im Körper gewährleistet.

Die Länge des gesamten Biopsie-Kits, umfassend das Führungselement, das Biofunktionselement und das Stabilisierungselement, ist so zu wählen, dass nach endoskopischer Platzierung, in der Regel über den Instrumentenkanal des Endoskops, das Endoskop aus dem Körper entfernt werden kann, ohne die Lage des Biopsie-Kits zu verändern. Dieses lässt sich dadurch bewerkstelligen, dass die Gesamtlänge des Biopsie-Kits an die Gesamtlänge des Endoskopes angepasst wird. Einem kundiger Fachmann ist es leicht möglich Sonderformen des Biopsie-Instrumentes zu konstruieren, bei denen zwei oder mehr Biofunktionselementen mit unterschiedlichen Detektionsmolekülen zwischen distalem und proximalem Anteil des Führungselementes angeordnet sind oder die Funktionalisierung des distalen Anteiles des Führungselementes mit Detektionsmolekülen vorzunehmen unter Erhalt der feder-elastische Materialeigenschaften dieser Region.

Vorteilhaft ist die Verwendung eines Biopsie-Kits, wenn das Biopsie-Instrument über einen Gefäßzugang in ein Gefäßsystem eingebracht wird. Hierbei können die auf dem Biofunktionselement angebrachten Detektionsmoleküle verschiedene Moleküle und/oder Zellen im Gefäßsystem detektieren. Für den Gebrauch des Biopsie-Kits über einen Gefäßzugang sind folgende Bestandteile als weitere Komponenten des Biopsie-Kits sinnvoll:
i. eine Blutgefäß-Verweilkanüle mit Punktionsnadel;
ii. ein Adapter, der sich an die Blutgefäß-Verweilkanüle anbringen und über den sich das Biopsie-Instrument intravasal positionieren lässt;
iii. einen Port, der vorzugsweise am Adapter ausgeführt ist und über den während der Verweilzeit des Biopsie-Instrumentes im Körper eine Infusionslösung appliziert werden kann;
iv. eine transparente und verschiebliche Hülle, die sich auf den Adapter aufschiebbar ist, das Biopsie-Instrument außerhalb des Körpers umgibt und eine hygienische Handhabung während seiner Verweilzeit im Körper gewährleistet.

Durch die vorteilhafte Ausführungsform kann schnell und effizient eine Probenanreicherung mit einer hohen Ausbeute erreicht werden, wenn das Biopsie-Kit in ein Gefäßsystem über einen Gefäßzugang eingeführt wird. Die gesammelte Probe kann nach Ausfuhr aus dem Gefäßsystem direkt analysiert werden oder an entsprechende Bearbeitungsstellen weitergeleitet werden. Die Ausführungsform stellt somit eine erhebliche Arbeitserleichterung für Krankenhauspersonal dar.

In sehr ähnlicher Weise kann das Biopsie-Kit zur Anreicherung und Sammlung von anstelle der Blutgefäß-Verweilkanüle eine Spinalkanal-Punktionskanüle bzw. eine Kanüle zur Punktion des Periduralraums genutzt wird, wie sie beispielsweise bei der Periduralanästhesie zum Einsatz kommt.

Neben der Platzierung über eine Blutgefäß-Verweilkanüle und via Endoskop ist auch eine Platzierung im Rahmen eines operativen Eingriffes oder im Rahmen einer minimal-invasiven chirurgischen Maßnahme vorteilhaft, was vorteilhafterweise an die Anforderungen bezüglich der Dimensionierung des Biopsie-Instrumentes keine zusätzlichen Anforderungen stellt als die bereits genannten. Die vorteilhafte Ausführungsform führt zu einer Verbesserung der Qualität und zu der Erhöhung der Zuverlässigkeit der Probenanreicherung.

Es ist bevorzugt, wenn das Biospie-Instrument und/oder das Kit zur Anreicherung von Proben verwendet wird und die Proben bevorzugt zur Diagnose insbesondere pränatalen Diagnose, Krebsdiagnose, der Therapie-Verlaufskontrolle (point of care Diagnostik) und/oder zur Diagnose der Homöostase von Patienten verwendet werden. Ferner lässt sich das Biopsie-Instrument vorteilhafterweise zur Diagnose einer Erkrankung ausgewählt aus der Gruppe umfassend Erberkrankungen, proliferativen Erkrankungen, inflammatorischen Erkrankungen, Autoimmunerkrankungen, Infektionserkrankungen, hormonellen Erkrankungen, Erkrankungen des Blutes und der blutbildenden Organe, Erkrankungen des Verdauungstraktes, der Leber, der Galle, der Bauchspeicheldrüse, Erkrankungen des Urogenitaltraktes und der Niere, Erkrankungen des Herzens, krankhafte Veränderungen des Blutgefäßsystems und des Lymphsystems, Erkrankungen der Lunge, Erkrankungen des zentralen oder peripheren Nervensystems sowie der elektrischen Reizweiterieitung und neurodegenerative Erkrankungen benutzen. Das Biopsie-Instrument sammelt vorteilhafterweise Proben, die zur Diagnose der genannten Krankheiten genutzt werden können, das heißt das Biopsie-Instrument dient der Erzeugung eines Zwischenschrittes. Das Biopsie-Instrument kann somit zur Diagnose von unterschiedlichen Erkrankungen herangezogen werden.

Bevorzugte Erbkrankheiten umfassen beispielsweise autosomal rezessive, autosomal dominante, gonosomale und mitochondriale beziehungsweise extrachromosomale Erberkrankung und/oder eine Erkrankung, die auf eine genetische Disposition zurück geführt werden kann. Proliferative Krankheiten umfassen bevorzugt Tumore, Präcancerose, Dysplasie, neuroendokrine Tumore, Endometriose und/oder Metaplasie.

Bevorzugte Autoimmunerkrankungen umfassen rheumatoide Arthritis, inflammatorische Darmerkrankung, Osteoarthritis, neuropathische Schmerzen, Alopecia areata, Psoriasis, psoriathrische Arthritis, akute Pankreatitis, Allograftabstoßung, Allergien, allergische Entzündungen der Lunge, Multiple Sclerose, Alzheimer, Morbus Crohn und/oder systemischer Lupus erythematous.

Infektionskrankheiten umfassen bevorzugt durch parasitäre Erkrankung, bakterielle Erkrankung und/oder viralen Erkrankungen ausgelöste Infekte.

Bevorzugte hormonelle Erkrankungen umfassen Erkrankung des Zuckerstoffwechsels, des Fettstoffwechsels, des Proteinstoffwechsels, der sexuellen Entwicklung und Fortpflanzung, des Wasser-Salz-Haushaltes, des Wachstums und/oder der Zellbildung.

Das Biopsie-Instrument besteht aus einem distalen feder-elastischen Führungselement und einem proximalen Stabilisierungselement, wobei zwischen dem distalen und proximalen Bereich ein oder mehrere Biofunktionselemente angebracht werden. Auf der Oberfläche von mindestens einem Biofunktionselement sind Detektionsmoleküle, umfassend Antikörper, Nukleinsäure oder sonstige dem Fachmann bekannte Fängerstrukturen aufgebracht. Es ist vorteilhaft, Sonderformen des Biopsie-Instrumentes zu konstruieren, bei denen zwei oder mehr Biofunktionselemente mit unterschiedlichen Detektionsmolekülen zwischen distalem und proximalem Anteil des Führungselementes angeordnet sind oder die Funktionalisierung des distalen Anteiles des Führungselementes mit Detektionsmolekülen vorzunehmen, unter Erhalt der feder-elastischen Materialeigenschaften dieser Region. Das proximal gelegene Stabilisierungselement dient der Stabilisierung des Biofunktionselemtes und verhindert dessen Dislokation bei der Einfuhr und Ausfuhr des Biopsie-Instrumentes. Der feder-elastischer Bereich des Führungselementes verhindert Verletzungen von biologischen Oberflächen bei der Einfuhr des Biopsie-Instrumentes in Körperregionen. Auf dem Biofunktionselement sind Detektionsmoleküle aufgebracht, die mit organischen und/oder anorganischen Materialien interagieren. Das Biofunktionselement kann entsprechend den Biopsie-Orten mit spezifischen Detektionsmolekülen ausgestattet werden, um so definierte Proben anzureichem. Die Verweildauer des Biopie-Instrumentes in den Körperregionen kann variiert werden und ist von den vorliegenden Konzentrationen des anzureichernden Materials abhängig. Das Biopsie-Instrument kann so in den Körperregionen positioniert werden, dass eine spezifische Anreicherung möglich ist. Weiterhin ist eine Kombination von Detektionsmolekülen möglich, womit eine Anreicherung unterschiedlicher Proben erreicht wird. Dies stellt einen Fortschritt dar, da so eine schnelle und verlässliche Diagnose und Therapie-Verlaufskontrolle möglich ist. Nach erfolgreicher Anreicherung der Probe wird das Biofunktionselement mittels einer Abdeckvorrichtung bedeckt, wodurch ein Verlust der Probe während der Entnahme aus dem Körper und eine Kontamination außerhalb des Körpers verhindert wird. Durch die Verwendung des Biopsie-Instrumentes werden Fehler bei der Probenentnahme verhindert und eine hohe Spezifität sichergestellt. Des Weiteren kann es für die Anreicherung von Proben verwendet werden, die in geringen Konzentrationen in schwer zugänglichen Körperregionen vorhanden sind.

Durch den einfachen und schnellen Austausch der Detektionsmoleküle des Biofunktionselementes oder des Biofunktionselementes, kann das Biopsie-Instrument in verschiedenen medizinischen Bereichen zur Diagnostik eingesetzt werden. Gerade bei der pränatalen und Tumor-Diagnostik ist eine schnelle und verlässliche Diagnostik entscheidend. Frühzeitig erkannt, können Krankheiten wie Herzfehler, Spina bifida und Lippen-Kiefer-Gaumenspalten therapiert werden. Somit bietet die Verwendung des Biopsie-Instrumentes mit den entsprechenden Detektionsmolekülen eine Möglichkeit, Proben anzureichern und diese auf Krankheiten zu analysieren und den Effekt einer eingeschlagenen Therapie zu überwachen, zu prüfen und gegebenenfalls zu ändern. Auch in dem Bereich der Krebsdiagnostik ist eine schnelle und zuverlässige Detektion unabdingbar, um frühzeitig therapeutische Behandlungen zu beginnen, zu überwachen und gegebenenfalls anzupassen. Hierfür muss jedoch der Tumor identifiziert werden. Die Verwendung des Biospie-Instrumentes offenbart die Möglichkeit, Krebsmarker, die charakteristisch für bestimmte Tumorarten sind, in Körperregionen, z.B. dem Gefäßsystem oder ableitenden Drüsengängen anzureichern und so eine schnelle Diagnose und Therapie-Verlaufskontrolle zu ermöglichen.

Vorteilhafterweise kann das Biopsie-Instrument zur Therapie-Verlaufskontrolle benutzt werden, das heißt es werden Proben mit dem Biopsie-Instrument gesammelt, die analysiert werden und der Kontrolle des Therapie-Verlaufs dienen. Diese umfasst die Überwachung einer Tumorbehandlung, die Überwachung einer autologen, syngenen, allogenen, xenogenen oder alloplastischen Transplantation, der Überwachung einer entzündlichen Erkrankung, der Überwachung einer Infektionserkrankung, der Überwachung einer hormonellen Erkrankung, der Überwachung einer psychiatrischen Erkrankung und/oder der Überwachung einer neurodegenerativen Erkrankung. Überraschenderweise kann das Biopsie-Instrument einfach und zuverlässig an die Diagnose von unterschiedlichen Krankheiten angepasst werden, indem ein oder mehrere Biofunktionselemente mit Fängerstrukturen versehen werden. Dem Fachmann sind diese Fängerstrukturen bekannt wodurch ebenfalls die Auswertung der gesammelten Proben bevorzugt automatisiert erfolgen kann. Die Proben sind demgemäß innerhalb kurzer Zeit ausgewertet und die gesammelten Daten können vorteilhafterweise zur Entscheidung über die Weiterbehandlung herangezogen werden.

Besonders vorteilhaft kann das Instrument zur Primärdiagnostik, zur Diagnostik der Tumorausbreitung oder zum Tumor Grading eingesetzt werden. Es können vorteilhafterweise Risikopatienten über einen längeren Zeitraum und in regelmäßigen Zeitintervallen Proben entnommen werden, um so ggf. eine Entstehung eines Tumors frühzeitig zu detektieren. Beispielsweise können die gesammelten Biopsie-Proben von einem Labor analysiert und ausgewertet werden. Jedoch kann das Instrument nicht nur zur Primärdiagnostik verwendet werden, sondern auch zur Langzeitüberwachung der Tumorausbreitung. Es kann beispielsweise das Tumorwachstum oder die Metastasenbildung eines Tumors überwacht werden. Diese Daten in Zusammenhang mit beispielsweise dem Differenzierungsgrad (Tumor Grading) des Tumors geben vorteilhafterweise dem behandelten Arzt wichtige Informationen, die für den weiteren Behandlungsverlauf entscheidend sein können. Vorteilhafterweise können die gesammelten Proben schnell und zuverlässig analysiert werden, was für eine erfolgreiche Behandlung ausschlaggebend ist.

Vorteilhafterweise kann das Biopsie-Instrument zur pränatalen Diagnose genutzt werden. Hierbei werden Proben gesammelt und bevorzugt auf Genmutationen, Chromosomenmutationen und Chromosomenaberrationen aus der Gruppe umfassend Deletion, Inversion, Duplikation, Translokation, Ringchromosomen, und/oder eine Störung der Gen-Transkription, der Gen-Translation, der mRNA-Stabilität, einer Splice-Varianten, einer Störung des mRNA Transportes ins Cytoplasma, der Protein-Biosynthese und/oder eines epigenetischen Faktors. Es können vorteilhafterweise Detektionsmoleküle, sogenannte Fängermoleküle, auf der Oberfläche eines oder mehrerer Biofunktionselemente angebracht werden. Die Moleküle sind bevorzugt spezifisch für die zu detektierenden Proben. Die gesammelten Proben können beispielsweise in einem Labor weitergehend analysiert werden und einem behandelnden Arzt zugehen. Vorteilhafterweise ist durch die Anreicherung von bevorzugt ausschließlich spezifischen Proben die Analyse wesentlich vereinfacht und in kürzerer Zeit durchführbar. Das Biopsie-Instrument ermöglicht nicht nur eine schnellere Analyse, sondern verbessert ebenfalls die Qualität der Proben. Es werden bevorzugt spezifische Proben gesammelt, die vorteilhafterweise bei dem Ausführen des Instrumentes aus dem Körper vor einer Dislokation geschützt sind.

Des Weiteren kann das Biopsie-Instrument dazu verwendet werden, die Homöostase von Patienten zu verfolgen. Gerade bei schwerwiegenden Krankheiten oder nach instrumentellen, chirurgischen Eingriffen ist eine kontinuierliche Überwachung des Patientenzustandes erforderlich. Das Biopsie-Instrument kann hierfür universell eingesetzt werden und vorteilhafterweise an die unterschiedlichen Anforderungen einfach, schnell und kostengünstig angepasst werden.

Weitere vorteilhafte Maßnahmen sind in den übrigen Unteransprüchen beschrieben.

Die Erfindung wird nunmehr anhand von Figuren beispielhaft beschrieben, ohne auf diese beschränkt zu sein. Es zeigen:
- Fig. 1: eine Seitenansicht eines Führungselementes mit einem distalen und proximalen Bereich,
- Fig. 2: eine Seitenansicht eines Führungselementes mit einem zylindrischen Biofunktionselement und einem zylindrischen Stabilisierungselement,
- Fig. 3: eine Seitenansicht eines Biopsie-Instrumentes, mit einer Aufnahmevorrichtung für ein halbzylindrisch-geformtes Biofunktionselement,
- Fig. 4: eine Seitenansicht eines Biopsie-Instrumentes, mit einer Aufnahmevorrichtung für zwei halbzylindrisch-geformte Biofunktionselement,
- Fig. 5: eine Seitenansicht eines Biopsie-Instrumentes, mit einer stegförmigen Aufnahmevorrichtung für ein spangenförmiggeformtes Biofunktionselement,
- Fig. 6: eine Seitenansicht eines Biopsie-Instrumentes, mit einer Aufnahmeeinrichtung für ein folienförmiges Biofunktionselement,
- Fig. 7: eine Seitenansicht eines Biopsie-Instrumentes mit einer Abdeckvorrichtung und
- Fig. 8: eine Seitenansicht eines Biopsie-Instrumentes mit einer Abdeckvorrichtung über einem Biofunktionselement.

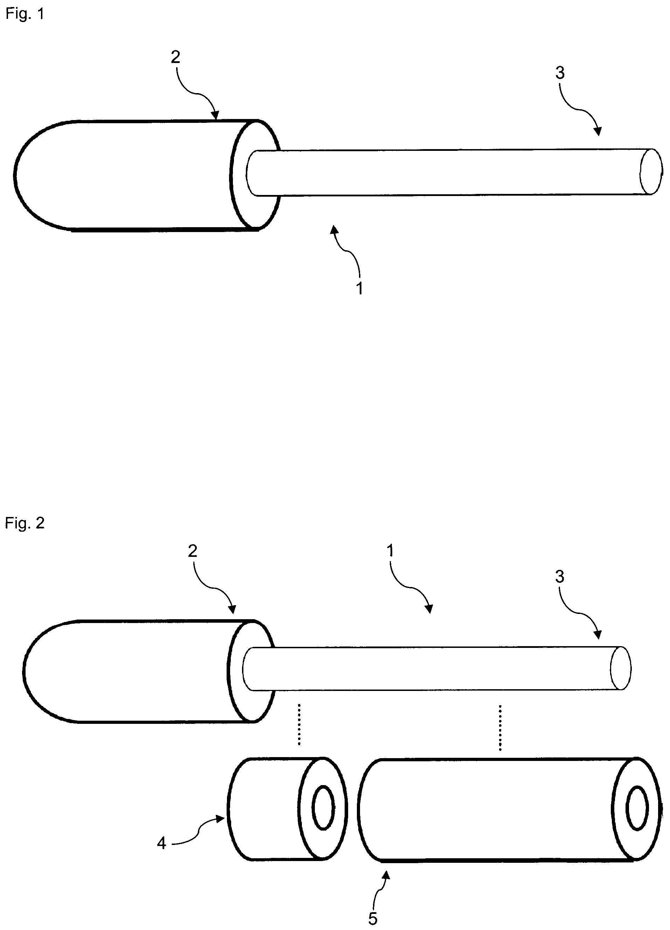

Fig. 1 zeigt eine schematische Seitenansicht eines Führungselementes. Das Führungselement 1 besteht aus einem distalen 2 und proximalen 3 Bereich. Der distale Bereich 2 des Führungselementes 1 ist feder-elastisch gestaltet. Er kann aus Metallen, vorzugsweise Stahl gefertigt sein. Alternativ können für diese Komponente aber auch nichtmetallische Materialien, aus denen sich dünne zylindrische Strukturen formen lassen, verwendet werden. Polymermaterialien wie z.B. Polytetrafluorethylen kommen hierfür in Frage. Der distale Bereich 2 des Führungselementes 1 ist fest mit dem proximalen Bereich 3 verbunden. Distaler 2 und proximaler 3 Bereich des Führungselementes 1 können aber auch in einem Herstellungsverfahren hergestellt werden, wenn das Material dieses erlaubt was beispielsweise durch den Einsatz geeigneter Kunststoffe bei der Herstellung möglich ist.

In Fig. 2 ist eine schematische Seitenansicht eines Führungselementes mit einem Biofunktionselement und einem Stabilisierungselement gezeigt. Das Biofunktionselement 4 in Fig. 2 kann Detektionsmoleküle auf seiner Oberfläche tragen, die mit spezifischen Zellen und/oder Molekülen im Körper von Mensch und Tier reagieren und dadurch eine Anreicherung von Probenmaterial *in situ* vor der Entnahme aus dem Körper gestatten. Die Ausgestaltung dieses Elementes ist im Wesentlichen als zylindrischer Hohlkörper auszuführen, der hinsichtlich seiner Abmessung so dimensioniert ist, dass er sich von dem proximalen Bereich 3 auf das Führungselement 1 aufbringen lässt. Hierzu ist der Innendurchmesser dieser Komponente größer als der Außendurchmesser des proximalen Bereiches 3 des Führungselementes 1. Der Außendurchmesser des Biofunktionselementes 4 in Relation zum distalen Bereich 2 des Führungselementes 1 kann größer, gleich oder kleiner sein. Vorzugsweise ist der Außendurchmesser des Biofunktionselementes 4 allerdings 0,01 bis 0,1 mm kleiner als der Außendurchmesser des distalen Bereichs 2 des Führungselementes 1. Materialien zur Herstellung dieser Komponente umfassen Metalle, Kunststoffe oder keramische Werkstoffe, die als Substrat der Verankerung von Detektionsmolekülen dienen können. Auch eine Ausgestaltung in Form von winzigen Hohlfasern oder Schwämmen ist möglich.

Des Weiteren ist in Fig. 2 ein Stabilisierungselement 5 dargestellt. Diese Komponente ist dadurch gekennzeichnet, dass sie rohrförmig ist und sich passgenau auf den proximalen Bereich 3 des Führungselementes 1 aufbringen lässt. Der Außendurchmesser des Stabilisierungselementes 5 entspricht dem Außendurchmesser des distalen Anteils 2 des Führungselementes 1. Die Wandstärke dieses rohrförmigen Elementes ist so dimensioniert, dass das Biofunktionselement 4 rutschfrei durch das Stabilisierungselement 5 auf dem Führungselement 1 fixiert wird. Das Stabilisierungselement 5 und der proximale Bereich 3 des Führungselementes 1 können dazu eine stabile Verbindung eingehen, was z.B. mechanisch, durch Verkleben oder durch Verschweißen dieser Elemente am proximalen Ende des Biopsie-Instrumentes ohne Funktionsverlust realisiert werden kann. Weiterhin ist vorteilhaft, wenn beiden Komponenten reversibel miteinander verbunden werden. Es kann so sichergestellt werden, dass nach erfolgreicher Probenanreicherung das Biofunktionselement von dem Biopsie-Instrument entfernt werden kann und zur Durchführung von nachgeschalteten Diagnose-Verfahren in die entsprechenden Labore versendet wird. Durch die Verwendung von Materialien für das Stabilisierungselement, die diesem gummi-elastischen Eigenschaften verleihen, können Biopsie-Instrumente hergestellt werden, die sich optimal an unterschiedliche Biopsie-Orte im Körper platzieren lassen.

Eine Ausführungsvariante des Biopsie-Instrumentes besteht darin, das Biofunktionselement 4 in Fig. 2 nicht nur in singulärer Form sondern in zwei bis mehrfacher Ausfertigung mit jeweils unterschiedlichen Detektionsmolekülen ausgestattet hintereinander auf dem proximalen Bereich 3 des Führungselementes 1 anzubringen, gefolgt von dem Stabilisierungselement 5.

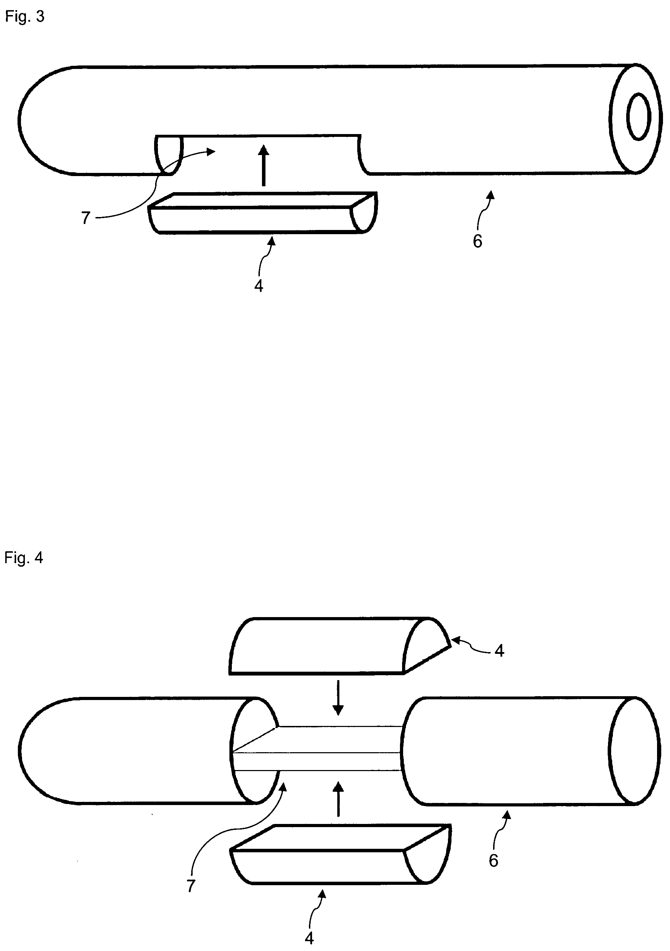

Fig.3 zeigt eine Seitenansicht eines Biopsie-Instrumentes, mit einer Aufnahmevorrichtung für ein halbzylindrisch-geformtes Biofunktionselement. Das Führungselement 1 und das Stabilisierungselement 5 können in einem Herstellungsverfahren gefertigt sein und dementsprechend permanent verbunden sein. Das so hergestellte Führungselement/Stabilisierungselement 6 weist eine Aufnahmevorrichtung 7 auf, die der Aufnahme eines Biofunktionselemtes 4 dient. Die Form der Aufnahmevorrichtung 7 kann unterschiedliche Formen aufweisen, wie z.B. die Form eines Halbzylinders. Das Biofunktionselement 4 ist entsprechend der Aufnahmevorrichtung 7 geformt. Dies ermöglicht die Variation der zu sammelnden Proben, oder ein schneller und einfacher Austausch des Biofunktionselementes 4, um dieses neuen Gegebenheiten anzupassen.

Fig. 4 stellt eine Seitenansicht eines Biopsie-Instrumentes, mit einer Aufnahmevorrichtung für zwei halbzylindrisch-geformte Biofunktionselemente dar. Das Biopsie-Instrument kann so gestaltet sein, dass das Führungselement 1 und das Stabilisierungselement 5 aus Fig. 2 in einem Herstellungsverfahren gefertigt sind, ein Führungselement/Stabilisierungselement 6 ergebend und eine Aufnahmevorrichtung 7 aufweisend in die zwei Biofunktionselemente 4 eingeführt werden können. Hierbei kann die Aufnahmevorrichtung 7 derart gestaltet sein, dass zweie halbzylindrisch-geformte Biofunktionselemente 4 in diese eingeführt werden können. Auf die beiden Biofunktionselemente 4 können unterschiedliche Detektionsmolekülen aufgebracht sein, die folglich die Anreicherung von unterschiedlichen Zellen und/oder Molekülen erlauben. Des Weiteren kann durch eine solche Formgestaltung die Ausbeute der Probe erhöht werden.

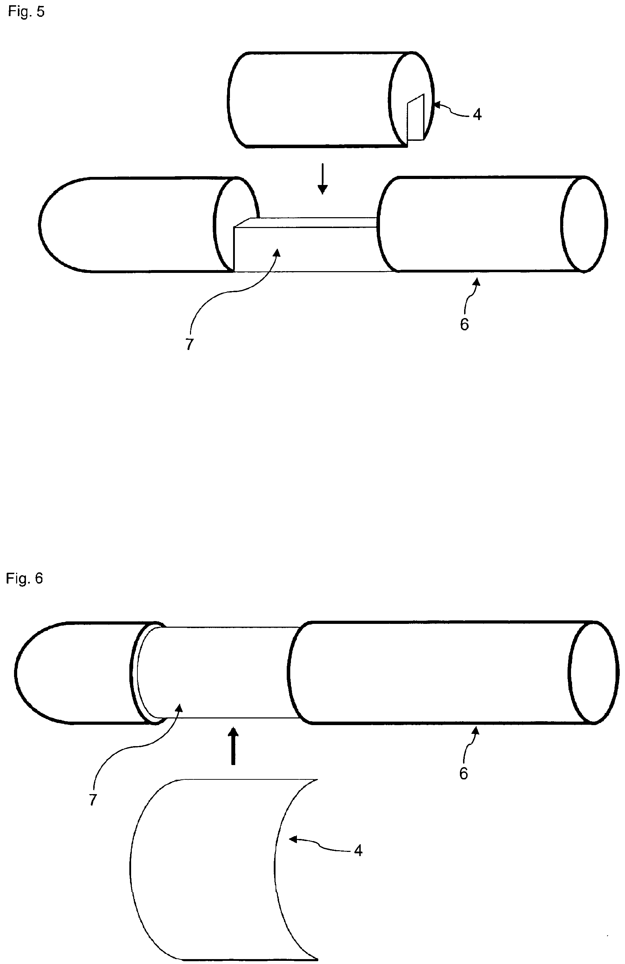

Fig. 5 zeigt eine Seitenansicht eines Biopsie-Instrumentes, mit einer stegförmigen Aufnahmevorrichtung für ein spangenförmig-geformtes Biofunktionselement Die Aufnahmevorrichtung 7 kann so gestaltet werden, dass sie stegförmig geformt ist und dementsprechend ein spangenförmiges Biofunktionselement 4 aufnehmen kann. Das so gestaltete Biofunktionselement 4 kann schnell ausgetauscht werden. Auch bei dieser Ausführungsalternative ist ein Biofunktionselement 4 zwischen einem distalen Bereich 2 und einem proximalen Stabilisierungselement 5 angeordnet. Es wird erreicht, dass bei minimaler Verletzungsgefahr für den Patienten die Biofunktion des Instrumentes gewährleistet bleibt und sich das Biopsie-Instrument nach Sammlung von Probenmaterial *in situ* in Gänze von außen aus dem Körper wieder entfernen lässt.

In Fig. 6 ist eine Seitenansicht eines Biopsie-Instrumentes, mit einer Aufnahmeeinrichtung für ein folienförmiges Biofunktionselement. Bei Anwendungen, die die Verminderung des Außendurchmessers des Biopsie-Instrumentes verlangen, kann eine folienförmige Aufnahmevorrichtung 7 zur Aufnahme eines folienförmigen Biofunktionselementes 4 dienen. Ein folienförmiges Biofunktionselement bezeichnet im Sinne der Erfindung eine Form mit einer sehr geringen Dicke und großer Fläche. Besonders in kleinen Gefäßen, in denen Proben angereichert werden müssen, kann diese Ausführungsform genutzt werden. Auch spezielle Detektionsmoleküle, in Form eines anorganischen Materials, mit denen die Anreicherung bestimmter organischer und/oder anorganischen Molekülen erreicht werden kann, können so in die Aufnahmevorrichtung 7 eingebracht werden. So kann das Biopsie-Instrument zur Anreicherung von Zellen und/oder Molekülen dienen, die in geringen Konzentrationen in Körperregionen vorliegen.

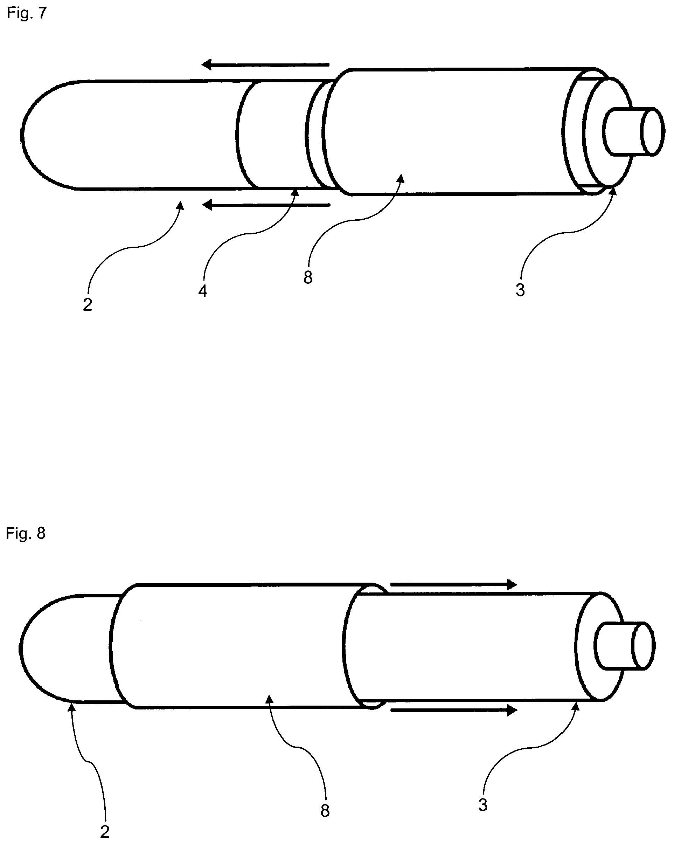

Fig. 7 zeigt eine Seitenansicht eines Biopsie-Instrumentes mit einer Abdeckvorrichtung. Hierbei ist eine Abdeckvorrichtung 8, die eine hülsenförmige Struktur umfassen kann und auf dem Biopsie-Instrument gleitet, dargestellt. Diese Abdeckvorrichtung 8 wird von dem proximalen Bereich des Führungselementes auf das Biopsie-Instrument aufgebracht. Als Materialien für die Abdeckvorrichtung 8 eignen sich Polymere, vorzugsweise Polytetrafluorethylen. Insbesondere wenn die angrenzenden Komponenten aus dem gleichen Material gefertigt sind, wird dadurch eine gute Verschiebbarkeit der Einzelkomponenten erreicht.

Fig. 8 stellt eine Seitenansicht eines Biopsie-Instrumentes mit einer Abdeckvorrichtung über einem Biofunktionselement dar. Hierbei ist die Abdeckvorrichtung 8 auf das Führungselement/Stabilisierungselement 6 aufgebracht und dient zur Abdeckung des Biofunktionselementes 4. Nach Anreicherung der Proben, wird die Abdeckvorrichtung in distalter Richtung über das Biofunktionselement 4 geschoben, wodurch das Biofunktionselement 4 und das angereicherte Probenmaterial bei der Ausfuhr des Biopsie-Instrumentes aus den Körperregionen geschützt und eine Kontamination vermieden wird. Es kann demgemäß eine hohe Qualität und Ausbeute erreicht werden.

### Bezugszeichenliste:

- 1: Führungselement
- 2: Distaler Bereich
- 3: Proximaler Bereich
- 4: Biofunktionselement
- 5: Stabilisierungselement
- 6: Führungselement/Stabilisierungselement
- 7: Aufnahmevorrichtung
- 8: Abdeckvorrichtung

## Patentansprüche

1. Ein Biopsie-Instrument zur Anreicherung von Probenmaterial,
**dadurch gekennzeichnet, dass**
das Biopsie-Instrument folgende Komponenten umfasst,
i. ein Führungselement (1), umfassend einen feder-elastischen distalen (2) und einen proximalen (3) Bereich,
ii. ein Biofunktionselement (4), welches zwischen dem distalen (2) und dem proximalen (3) Bereich des Führungselementes (1) angebracht ist, und dessen Oberfläche Detektionsmoleküle aufweist, und
iii. ein mit dem proximalen Bereich (3) des Führungselementes (1) verbundenes Stabilisierungselement (5).

2. Biopsie-Instrument nach Anspruch 1,
**dadurch gekennzeichnet, dass**
die Komponenten von distal nach proximal sequentiell angeordnet sind.

3. Biopsie-Instrument nach einem oder mehreren der vorhergehenden Ansprüche,
**dadurch gekennzeichnet, dass**
das Biofunktionselement (4) einen Außendurchmesser hat, der größer, gleich oder kleiner, vorzugsweise 0,01 bis 0,1 mm kleiner als der Außendurchmesser des distalen Bereichs (2) des Führungselementes (1) ist.

4. Biopsie-Instrument nach einem oder mehreren der vorhergehenden Ansprüche,
**dadurch gekennzeichnet, dass**
mehr als ein Biofunktionselement (4) mit jeweils unterschiedlichen Detektionsmolekülen simultan zum Einsatz kommt.

5. Biopsie-Instrument nach einem oder mehreren der vorhergehenden Ansprüche,
**dadurch gekennzeichnet, dass**
das Führungselement (1) zwischen dem distalen (2) und proximalen (3) Bereich eine Aufnahmevorrichtung (7) aufweist, welche der Aufnahme des Biofunktionselementes (4) dient.

6. Biopsie-Instrument nach einem oder mehreren der vorhergehenden Ansprüche,
**dadurch gekennzeichnet, dass**
das Biofunktionselement (4) Metall, Kunststoff, und/oder keramischen Werkstoffen umfasst.

7. Biopsie-Instrument nach einem oder mehreren der vorhergehenden Ansprüche,
**dadurch gekennzeichnet, dass**
der distale Bereich (2) des feder-elastischen Führungselementes (1) Detektionsmoleküle aufweist.

8. Biopsie-Instrument nach einem oder mehreren der vorhergehenden Ansprüche,
**dadurch gekennzeichnet, dass**
es eine longitudinale Bohrung aufweist, zur Aufnahme bevorzugt eines Mandrains und/oder anderer Funktionselemente.

9. Biopsie-Kit umfassend ein Biopsie Instrument nach einem oder mehreren der vorhergehenden Ansprüche, umfassend . ein oder mehrere Biofunktionselemente (4), welche zwischen dem distalen (2) und dem proximalen (3) Bereich des Führungselement (1) angebracht sind, wobei auf der Oberfläche von mindestens einem Biofunktionselement (4) Detektionsmoleküle aufgebracht sind,
wobei das Führungselement (1), das Biofunktionselement (4) und das Stabilisierungselement (5) sequentiell von distal nach proximal angeordnet sind.

## Claims

1. A biopsy instrument for enriching sample material,
**characterized by**
comprising the following components:
i. a guide element (1) comprising a spring-elastic distal portion (2) and a proximal portion (3);
ii. a biofunction element (4), which is provided between the distal portion (2) and the proximal portion (3) of the guide element (1) and the surface of which includes detection molecules; and
iii. a stabilizing element (5) connected to the proximal portion (3) of the guide element (1).

2. The biopsy instrument according to claim 1,
**characterized in that**
the components are sequentially arranged from distal to proximal.

3. The biopsy instrument according to one or more of the preceding claims,
**characterized in that**
the biofunction element (4) has an outside diameter that is greater than, equal to or smaller than, preferably 0.01 to 0.1 mm smaller than, the outside diameter of the distal portion (2) of the guide element (1).

4. The biopsy instrument according to one or more of the preceding claims,
**characterized in that**
more than one biofunction element (4) is used at a time, each having different detection molecules.

5. The biopsy instrument according to one or more of the preceding claims,
**characterized in that**
the guide element (1) comprises a receiving device (7) between the distal portion (2) and the proximal portion (3), which serves to receive the biofunction element (4).

6. The biopsy instrument according to one or more of the preceding claims,
**characterized in that**
the biofunction element (4) comprises metal, plastic, and/or ceramic materials.

7. The biopsy instrument according to one or more of the preceding claims,
**characterized in that**
the distal portion (2) of the spring-elastic guide element (1) includes detection molecules.

8. The biopsy instrument according to one or more of the preceding claims,
**characterized by**
having a longitudinal bore for receiving preferably a mandrain and/or other functional elements.

9. A biopsy kit comprising a biopsy instrument according to one or more of the preceding claims, comprising one or more biofunction elements (4), which are provided between the distal portion (2) and the proximal portion (3) of the guide element (1), wherein detection molecules are applied to the surface of at least one biofunction element (4), and the guide element (1), the biofunction element (4) and the stabilizing element (5) are sequentially arranged from distal to proximal.

## Revendications

1. Instrument de biopsie pour l'enrichissement d'un matériau échantillon, **caractérisé en ce que** l'instrument de biopsie comprend les composants suivants :
I. un élément de guidage (1) comprenant une zone distale élastique (2) et une zone proximale (3),
II. un élément biofonctionnel (4) aménagé entre la zone distale (2) et la zone proximale (3) de l'élément de guidage (1) et dont la surface présente des molécules de détection, et
III. un élément de stabilisation (5) relié à la zone proximale (3) de l'élément de guidage (1).

2. Instrument de biopsie selon la revendication 1, **caractérisé en ce que** les composants sont disposés séquentiellement de la partie distale à la partie proximale.

3. Instrument de biopsie selon une ou plusieurs des revendications précédentes, **caractérisé en ce que** l'élément biofonctionnel (4) présente un diamètre extérieur qui est supérieur, égal ou inférieur, de préférence inférieur de 0,01 à 0,1 mm, au diamètre extérieur de la zone distale (2) de l'élément de guidage (1).

4. Instrument de biopsie selon une ou plusieurs des revendications précédentes, **caractérisé en ce que** plus d'un élément biofonctionnel (4) avec des molécules de détection respectivement différentes est mis en oeuvre simultanément.

5. Instrument de biopsie selon une ou plusieurs des revendications précédentes, **caractérisé en ce que** l'élément de guidage (1) entre la zone distale (2) et la zone proximale (3) présente un dispositif de réception (7) servant à recevoir l'élément biofonctionnel (4).

6. Instrument de biopsie selon une ou plusieurs des revendications précédentes, **caractérisé en ce que** l'élément biofonctionnel (4) comprend du métal, de la matière plastique et/ou des matières céramiques.

7. Instrument de biopsie selon une ou plusieurs des revendications précédentes, **caractérisé en ce que** la zone distale (2) de l'élément de guidage (1) élastique présente des molécules de détection.

8. Instrument de biopsie selon une ou plusieurs des revendications précédentes, **caractérisé en ce qu'**il présente un perçage longitudinal pour recevoir de préférence un mandrin et/ou d'autres éléments fonctionnels.

9. Kit de biopsie, comprenant un instrument de biopsie selon une ou plusieurs des revendications précédentes, comprenant un ou plusieurs éléments biofonctionnels (4) aménagés entre la zone distale (2) et la zone proximale (3) de l'élément de guidage (1), dans lequel des molécules de détection sont appliquées à la surface d'au moins un élément biofonctionnel (4), dans lequel l'élément de guidage (1), l'élément biofonctionnel (4) et l'élément de stabilisation (5) sont disposés séquentiellement de la partie distale à la partie proximale.
